# EUROPÄISCHE PATENTSCHRIFT

(11) **EP 1 281 359 B1**
(45) Veröffentlichungstag und Bekanntmachung des Hinweises auf die Patenterteilung: **26.11.2008**
(21) Anmeldenummer: 02013682.6
(22) Anmeldetag: 01.07.2002
(51) Int. Cl.: A61B 17/322, A61B 17/32

(54) **Chirurgisches Messer**
Surgical knife
Couteau chirurgical

(30) Priorität: 02.08.2001 DE 10137916
(43) Veröffentlichungstag der Anmeldung: 05.02.2003
(73) Patentinhaber: Aesculap AG, 78532 Tuttlingen (DE)
(72) Erfinder: Dworschak, Manfred, 78589 Dürbheim (DE); Lutze, Theodor, 78582 Balgheim (DE); Morales, Pedro, 78532 Tuttlingen-Nendingen (DE); Weisshaupt, Dieter, 78194 Immendingen (DE)
(74) Vertreter: Hoeger, Stellrecht & Partner Patentanwälte

(56) Entgegenhaltungen:
- DE-A- 2 935 141
- DE-A- 3 826 786
- US-A- 3 732 869
- US-A- 4 221 222
- US-A- 4 709 481

## Beschreibung

Die Erfindung betrifft ein chirurgisches Messer mit einem Klingenhalter und einer Klinge.

Solche chirurgischen Messer werden beispielsweise als Skalpelle oder Schälmesser eingesetzt.

Aus der DE 29 35 141 A1 ist ein Schneidwerkzeug aus Plast für weiches Material bekannt, welches wenigstens im Schneidenbereich mit einer Spritzschicht aus einem harten Material beschichtet ist.

Der Erfindung liegt die Aufgabe zugrunde, ein chirurgisches Messer zu schaffen, welches kostengünstig herstellbar ist, um es insbesondere als Einweginstrument einzusetzen.

Diese Aufgabe wird bei einem chirurgischen Messer der eingangs genannten Art erfindungsgemäß dadurch gelöst, daß die Klinge aus einem faserverstärkten Kunststoffmaterial hergestellt ist.

Versuche haben ergeben, daß eine aus einem faserverstärkten Kunststoffmaterial hergestellte Klinge genügend scharf ist, um in Gewebe Schnitte einbringen zu können. Dieser Effekt ist vermutlich darauf zurückzuführen, daß sich, wenn faserverstärkter Kunststoff (gefüllter Kunststoff) thermisch in eine Form gefüllt, d. h. in diese und in dieser strömt, die Fasern im Bereich einer Schneidkante der Klinge quer zu dieser ausrichten und dadurch eine Welligkeit an der Schneidkante verursachen, welche die gleiche Wirkung hat wie ein Wellenschliff bei einer herkömmlichen metallischen Klinge. Aufgrund der Faserstruktur läßt sich dann aber auch eine sehr scharfe Schneidkante ausbilden, so daß entsprechend die Schneidfläche klein ist und sich damit hohe Drücke auf Gewebe ausüben lassen. Die Fasern selber sorgen dabei für eine Verstärkung des Kunststoffs, d. h. erhöhen dessen Härte, so daß das faserverstärkte Kunststoffmaterial die hohen Kräfte auch aufnehmen kann, welche es selber auf Gewebe ausübt. Versuche haben gezeigt, daß die Schnittwirkung ausreichend ist.

Ein besonderer Vorteil einer aus einem faserverstärkten Kunststoffmaterial hergestellten Klinge ist, daß sich diese einstückig mit dem Klingenhalter ausbilden läßt, so daß sich ein chirurgisches Messer integral in demselben Arbeitsgang herstellen läßt. Solch ein chirurgisches Messer ist damit kostengünstig herstellbar und damit ideal für den Einsatz als Einweginstrument.

Günstigerweise ist also die Klinge einstückig an dem Klingenhalter angeordnet, d. h. bei der Herstellung werden Klingenhalter und Klinge integral hergestellt.

Vorteilhafterweise ist der Klingenhalter an einem Haltegriff angeordnet, um entsprechend die Klinge zur Durchführung einer Schneidbewegung führen zu können. Der Haltegriff kann dabei auf lösbare Weise mit dem Klingenhalter verbunden werden, beispielsweise über eine Schraubenverbindung.

Auf kostengünstige Weise läßt sich ein Vollkunststoff-Messer herstellen, wenn der Handgriff und der Klingenhalter einstückig ausgebildet ist und die Klinge wiederum einstückig an dem Klingenhalter sitzt. Es läßt sich dann integral ein komplettes chirurgisches Messer über beispielsweise ein Kunststoff-Spritzgußverfahren herstellen. Eine Klinge muß dann nachträglich nicht mehr an dem Klingenhalter fixiert werden. Aufgrund des Wegfallens dieses Arbeitsgangs ergeben sich verringerte Herstellungskosten.

Ganz besonders günstig ist es, wenn die Klinge an einer Schneidkante keilförmig ausgebildet ist, um die Fläche, mit der die Klinge an der Schneidkante auf Gewebe wirken kann, gering zu halten und damit hohe Anpreßdrücke zu erzeugen. Es hat sich als vorteilhaft erwiesen, wenn ein Keilwinkel im Bereich zwischen 10° und 30° liegt und insbesondere zwischen 15° und 25° liegt. Es wurden dabei praktische Versuche mit Keilwinkeln von ca. 20° durchgeführt, bei denen sich hervorragende Ergebnisse eingestellt haben.

Zur Erzielung einer guten Schneidwirkung weist eine Schneidkante der Klinge eine Welligkeit auf, die in der Art eines Wellenschliffs wirkt und über die sich insbesondere dann mit hoher Schneidwirkung ein ziehender Schnitt in Gewebe einbringen läßt, beispielsweise zum Entfernen von Rachenmandeln.

Hergestellte und auf ihre Schneidwirkung positiv getestete Klingen weisen dabei eine mittlere Wellentiefe im Bereich zwischen 0,03 mm und 0,16 mm auf, wobei insbesondere eine mittlere Wellentiefe im Bereich zwischen 0,06 mm und 0,10 mm liegt.

Ferner ist es günstig, wenn ein mittlerer Wellenabstand quer zu der Abstandsrichtung zur Ermittlung der Wellentiefe im Bereich zwischen 0,05 mm und 0,25 mm liegt und insbesondere im Bereich zwischen 0,1 mm und 0,2 mm liegt.

Weiterhin hat es sich als günstig erwiesen, wenn der Faseranteil im Kunststoffmaterial im Bereich zwischen 25 % und 70 % liegt und insbesondere im Bereich zwischen 40 % und 60 % liegt. Sehr gute Ergebnisse haben sich erzielen lassen mit teilaromatisiertem PA als Kunststoffmaterial mit einem sechzigprozentigen Anteil an Glasfaser. Als ebenfalls geeignet erwiesen hat sich LCP als Kunststoffmaterial mit eine dreißigprozentigen Anteil an Karbonfasern.

Bei den Fasern zur Verstärkung des Kunststoffs, d. h. zur Erzielung dessen höherer Härte, kann es sich dabei um Glasfasern oder alternativ (oder auch zusätzlich) um Kohlenstoffasern handeln.

Zur Erzielung einer Schneidwirkung ist es günstig, wenn die Fasern des Kunststoffmaterials an einer Schneidkante der Klinge im statistischen Mittel quer zur Schneidkante ausgerichtet sind. Dadurch ergibt sich zum einen eine hohe Härte quer zur Schneidrichtung, da die Fasern eine hohe Biegesteifigkeit in ihrer Längsrichtung aufweisen. Zum anderen läßt sich dadurch die Schneidkante mit einer geringen Fläche ausbilden, um so bei entsprechender Kraftausübung einen hohen Anpreßdruck auf Gewebe erzeugen zu können.

Weiterhin ist es günstig, wenn die Fasern des Kunststoffmaterials in der Klinge außerhalb eines Schneidkantenbereichs im statistischen Mittel parallel oder in einem kleinen Winkel zu der Schneidkante ausgerichtet sind. Die Kunststoffasern in einem solchen zweiten Bereich, welcher sich an den ersten Bereich an der Schneidkante anschließt, können dadurch die Fasern in dem ersten Bereich halten und ein Zurückweichen in die Klinge hinein bei Kraftausübung zumindest verringern. Sie stellen also in gewissem Maß ein Haltebett für die "Schneidfasern" in dem Bereich an der Schneidkante dar.

Günstigerweise ist dabei die Klinge in einer Form hergestellt, welche zur Herstellung der Schneidkante im wesentlichen auf Null ausläuft, um so eine kleine Fläche zur Sicherung hoher Anpreßdrücke an der Schneidkante bereitzustellen.

Ferner ist es günstig, wenn die Klinge so hergestellt ist, daß flüssiger Kunststoff so in einer Form geführt ist, daß sich Fasern an einer Schneidkante quer zu dieser ausrichten, um entsprechend die Wellung zur Herstellung der Schneidwirkung zu bilden. Die Strömungsverhältnisse sollten in der Form so sein, daß sich im Bereich der herzustellenden Schneidkante die Fasern also quer ausrichten können. Ferner ist es dabei vorteilhaft, wenn außerhalb dieses Bereichs der Schneidkante sich die Fasern parallel oder in einem kleinen Winkel zu dieser ausrichten können.

Mikroskopische Schnittuntersuchungen haben ergeben, daß eine mittlere Faserlänge im Bereich der Klinge an der Schneidkante kürzer ist als eine mittlere Faserlänge außerhalb des Bereichs der Schneidkante. Insbesondere kann eine mittlere Faserlänge im Bereich der Schneidkante zwischen 20 µm und 50 µm liegen. Eine mittlere Faserlänge außerhalb des Bereichs der Schneidkante kann zwischen 70 µm und 200 µm liegen. Es scheint günstiger zu sein, wenn die Fasern an der Schneidkante, welche die Kräfte auf das Gewebe ausüben, kürzer sind, um die Quersteifigkeit der Klinge an der Schneidkante zu erhöhen. Die Fasern in dem Bereich außerhalb des Schneidkantenbereichs können dann als eine Art von Haltebett für die Fasern im Schneidkantenbereich dienen, um bei einer Kraftausübung auf die Schneidkante deren Ausweichen durch Verformung zu verringern.

Bei einer Variante einer Ausführungsform ist das chirurgische Messer als Schälmesser ausgebildet, mit dem sich in Gewebe ein ziehender Schnitt einbringen läßt. Solch ein Schälmesser wird beispielsweise zur Entfernung von Rachenmandeln eingesetzt.

Insbesondere ist dabei die Klinge quer zu einer Längsrichtung eines Haltegriffs orientiert, wobei der Klingenhalter im Bereich seines Halteendes so bogenförmig ausgebildet ist, daß die Klinge versetzt zu einer Längsebene des Haltegriffs angeordnet ist. Das derart ausgebildete Schälmesser läßt sich dann in eine Körperöffnung einführen, an dem zu schneidenden Gewebe positionieren und durch einen ziehenden Schnitt, der beispielsweise über eine Art Schwenkbewegung einbringbar ist, läßt sich dann ein entsprechendes Gewebestück entfernen.

Insbesondere ist dabei eine Schneidkante der Klinge einem Ende des Klingenhalters abgewandt angeordnet, um durch eine Zubewegung auf den Operateur einen Schnitt einbringen zu können.

Bei dem Kunststoffmaterial handelt es sich insbesondere um einen thermoplastischen Kunststoff wie beispielsweise PEEK, LCP oder PA.

Die nachfolgende Beschreibung eines bevorzugten Ausführungsbeispiels dient im Zusammenhang mit der Zeichnung der näheren Erläuterung der Erfindung.
Es zeigen:
- Figur 1: eine Draufsicht in Teilschnittdarstellung auf ein Schälmesser als ein Ausführungsbeispiel eines chirurgischen Messers;
- Figur 2: eine seitliche Schnittansicht längs der Linie 2-2 gemäß Figur 1;
- Figur 3: eine vergrößerte Teilansicht einer Klinge des Schälmessers von Figur 1;
- Figur 4: eine vergrößerte seitliche Darstellung der Klinge;
- Figur 5: eine schematische Seitenansicht einer Form zur Herstellung der Klinge gemäß Figur 4 und
- Figur 6: eine vergrößerte Ausschnittansicht längs der Linie 6-6 gemäß Figur 4 resultierend aus einer mikroskopischen Längsschliffuntersuchung.

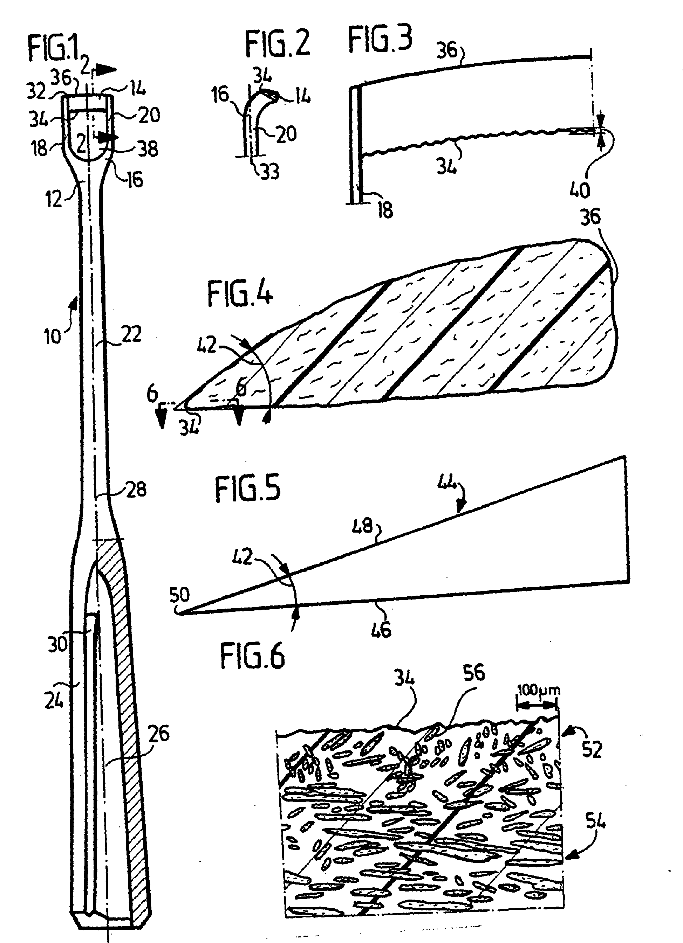

Ein erfindungsgemäßes chirurgisches Messer, von dem in Figur 1 als Ausführungsbeispiel ein dort als Ganzes mit 10 bezeichnetes Schälmesser gezeigt ist, umfaßt einen Klingenhalter 12, an dem eine Klinge 14 angeordnet ist. Der Klingenhalter 12 weist dabei ein gabelförmig ausgestaltetes Halteteil 16 auf mit einem ersten Zinken 18 und einem gegenüberliegenden zweiten Zinken 20. Im Bereich der Zinken 18 und 20 ist das Halteteil 16 U-förmig ausgestaltet.

An das Halteteil 16 anschließend weist der Klingenhalter 12 ein Verbindungsteil 22 auf, welches einstückig mit dem Halteteil 16 verbunden ist.

Mit dem Verbindungsteil 22 wiederum ist ein Haltegriff 24 verbunden, über welchen ein Operateur das Schälmesser 10 halten und führen kann.

Der Klingenhalter 12 ist aus einem Kunststoffmaterial hergestellt. Der Haltegriff 24 kann ebenfalls aus einem Kunststoffmaterial hergestellt sein und einstückig mit dem Klingenhalter 12 verbunden sein. Insbesondere weist er dann einen Hohlkern 26 zur Materialersparnis und gegebenenfalls zur leichteren Herstellbarkeit auf.

Der Haltegriff 24 kann aber auch über eine lösbare Verbindung und insbesondere über eine Schraubverbindung mit dem Klingenhalter 12 verbunden sein. Der Klingenhalter 12 läßt sich dann über das Verbindungsteil 22 mit dem Haltegriff 24 verbinden, und nach Gebrauch lassen sich dann diese beiden Teile voneinander lösen und der Haltegriff 24 kann weiter verwendet werden. Bei dieser Variante kann der Haltegriff 24 auch aus einem metallischen Material hergestellt sein.

Der Haltegriff 24 und das Verbindungsteil 22 weisen eine Längsrichtung 28 auf, um die insbesondere das Verbindungsteil 22 rotationssymmetrisch ausgebildet ist. Der Haltegriff 24 ist ebenfalls rotationssymmetrisch um die Längsrichtung 28, abgesehen von eventuellen Griffrillen 30, ausgebildet.

Das Halteteil 16 ist bogenförmig ausgebildet, so daß ein vorderes Ende 32 versetzt zu einer durch die Längsrichtung 28 definierten Längsebene 33 des Haltegriffs 24 und des Verbindungsteils 22 versetzt ist, d. h. das vordere Ende 32 weist einen Abstand zu dieser Längsebene 33 auf und damit auch die Klinge 14 und insbesondere eine Schneidkante 34 der Klinge 14 (Figur 2).

Die Klinge 14 ist zwischen dem ersten Zinken 18 und dem zweiten Zinken 20 angeordnet, wobei ein vorderes, stumpfes Ende 36 der Klinge 14 im wesentlichen bündig mit dem vorderen Ende 32 des Klingenhalters 12 abschließt. Ein dem stumpfen Ende 36 abgewandtes Ende 34 bildet die Schneidkante 34, welche quer zu der Längsachse 28 parallel zur Längsebene 33 orientiert ist.

Zwischen der Schneidkante 34 der Klinge 14 und den beiden Zinken 18 und 20 weist der Klingenhalter 12 einen Freiraum 38 auf, welcher durch eine durchgehende Ausnehmung gebildet ist und über die ein Operationsgebiet zur Durchführung eines ziehenden Schnitts zum Operateur hin zugänglich ist.

Ein solches Schälmesser 10 wird aufgrund der bogenförmigen Ausbildung des Klingenhalters 12 auch als Ringmesser bezeichnet.

Die Klinge 14 ist aus einem faserverstärkten thermoplastischen Kunststoffmaterial wie beispielsweise faserverstärktem teilaromatisiertem PA, LCP, PEEK, PP, PROM oder einem anderen für chirurgische Instrumente zulässigen Material gefertigt und insbesondere einstückig mit dem Klingenhalter 12 gebildet. Bei der Herstellung eines solchen Schälmessers 10 wird also die Klinge 14 in der gleichen Form zusammen mit dem Klingenhalter 12 hergestellt.

Durch die Faserverstärkung erhöht sich die Härte der Klinge 14. Es kann sich dabei um Glasfasern oder Karbonfasern handeln ("gefüllter" Kunststoff). Der Anteil der Glasfasern liegt dabei im Bereich zwischen 30 % und 60 % und insbesondere im Bereich zwischen 40 % bis 60 %.

Ein bevorzugtes Ausführungsbeispiel wurde aus teilaromatisiertem PA mit einem sechzigprozentigen Anteil an Glasfasern hergestellt.

Ein weiteres Instrument wurde aus LCP mit einem dreißigprozentigen Anteil an Kohlenstoffasern hergestellt.

Wie in Figur 3 gezeigt, weist die Schneidkante 34 eine Welligkeit auf, die insbesondere durch die Herstellung der Klinge 14 erreicht ist und nicht durch nachträgliche Bearbeitung. Diese Welligkeit entspricht von der Funktion her einem Wellenschliff bei einer metallischen Klinge. Es hat sich dabei in der Praxis gezeigt, daß sich gute Schneidergebnisse erzielen lassen, wenn eine mittlere Wellentiefe, ein Abstand 40 zwischen einem Punkt, welcher den größten Abstand zu dem stumpfen Ende 36 aufweist und einem Punkt, welcher den geringsten Abstand zu dem stumpfen Ende 36 aufweist, im Bereich zwischen 0,03 mm und 0,16 mm liegt und insbesondere bei ca. 0,08 mm liegt.

Ferner hat es sich als günstig erwiesen, wenn ein mittlerer Wellenabstand, d. h. ein Abstand quer zu einer Abstandsrichtung zwischen dem stumpfen Ende 36 und der Schneidkante 34, zwischen ca. 0,05 mm und 0,25 mm und insbesondere zwischen ca. 0,1 mm und 0,2 mm liegt.

Die Klinge 14 ist im Bereich der Schneidkante 34 keilförmig ausgebildet (Figur 4) mit einem Keilwinkel 42, welcher im Bereich zwischen 15° und 25° liegt und insbesondere bei ca. 20° liegt. Eine solche Klinge 14 läßt sich, wie in Figur 5 gezeigt, durch eine Form 44 herstellen, welche im Bereich der Klinge 14 ebenfalls keilförmig ausgestaltet ist und bei der entsprechende Begrenzungsflächen 46, 48 in einem spitzen Winkel entsprechend dem Keilwinkel 42 aufeinanderstoßen, d. h. am Stoß 50 auf Null auslaufen; am Stoß 50 ist dann der Abstand zwischen den Begrenzungsflächen 46 und 48 im wesentlichen Null.

Mikroskopische Untersuchen haben gezeigt (Figur 6), daß die aus dem faserverstärkten Kunststoff hergestellte Klinge 14 unterschiedliche Bereiche 52, 54 aufweist: In einem ersten Bereich 52, welcher durch die Schneidkante 24 abgeschlossen ist, sind die Fasern 56 des faserverstärkten (fasergefüllten) Kunststoffs im statistischen Mittel quer in einem Winkel näher zu 90° als zu 0° zur Schneidkante 34 orientiert und sind kürzer als in einem zweiten Bereich 54, welcher sich an den ersten Bereich 52 in Richtung Inneres der Klinge 14 anschließt. In diesem zweiten Bereich 54 sind die Fasern 56 im statistischen Mittel parallel oder in einem kleinen Winkel (deutlich näher zu 0° als zu 90°) zu der Schneidkante 34 orientiert und im Mittel länger als im ersten Bereich 52.

Für eine Klinge 14, welche aus teilaromatisiertem PA mit einem sechzigprozentigen Glasfaseranteil hergestellt ist, konnte eine Ausdehnung des ersten Bereichs von der Schneidkante 34 weg von ca. 200 µm bei der mikroskopischen Untersuchung von Längsschliffen ermittelt werden. Eine mittlere Faserlänge in diesem Bereich liegt dabei unterhalb von 100 µm, während im zweiten Bereich 54 eine mittlere Faserlänge im Bereich von ca. 100 µm oder höher liegt.

Die Querorientierung der Fasern 56 in dem ersten Bereich 52 verursacht die Welligkeit der Schneidkante 34, wodurch wiederum eine gute Schneidwirkung der Klinge 14 hergestellt wird, so daß das Schälmesser 10 eine hervorragende Schneidwirkung aufweist.

Vorzugsweise wird deshalb die Klinge 14 so hergestellt, daß beim Einfüllen von flüssigem Kunststoff in die Form 44 die Strömung des flüssigen Kunststoffs derart ausgebildet ist, daß sich die Fasern im Bereich des Stoßes 50 zur Bildung der Schneidkante 34 quer ausrichten können.

Das erfindungsgemäße Schälmesser 10 läßt sich einstückig durch Vergießen von faserverstärktem Kunststoff (gefülltem Kunststoff) herstellen. Auf diese Weise läßt sich das Schälmesser 10 integral ausbilden, ohne daß weitere Teile an diesem noch angeordnet werden müssen. Es läßt sich dadurch als Einmalinstrument (Wegwerfinstrument) einsetzen, da die Herstellungskosten insbesondere bei einer großen Stückzahl erniedrigt sind.

Ein erfindungsgemäßes chirurgisches Messer mit einer Klinge aus einem faserverstärkten Kunststoffmaterial kann beispielsweise auch als Skalpell oder dergleichen ausgebildet sein, wobei dann eine Schneidkante beispielsweise parallel zu einer Längsrichtung eines Haltegriffs angeordnet ist (in der Zeichnung nicht gezeigt).

Es kann auch vorgesehen sein, daß eine entsprechende Klinge als separates Teil hergestellt wird, welches dann anschließend mit einem Klingenhalter verbunden wird.

## Patentansprüche

1. Chirurgisches Messer mit einem Klingenhalter (12) und einer Klinge (14), welche aus einem faserverstärkten Kunststoffmaterial hergestellt ist.

2. Chirurgisches Messer nach Anspruch 1, **dadurch gekennzeichnet, daß** die Klinge (14) einstückig an dem Klingenhalter (12) angeordnet ist.

3. Chirurgisches Messer nach Anspruch 1 oder 2, **dadurch gekennzeichnet, daß** der Klingenhalter (12) an einem Haltegriff (24) angeordnet ist.

4. Chirurgisches Messer nach Anspruch 3, **dadurch gekennzeichnet, daß** der Haltegriff (24) und der Klingenhalter (12) einstückig ausgebildet sind.

5. Chirurgisches Messer nach einem der vorangehenden Ansprüche, **dadurch gekennzeichnet, daß** die Klinge (14) an einer Schneidkante (34) keilförmig ausgebildet ist.

6. Chirurgisches Messer nach Anspruch 5, **dadurch gekennzeichnet, daß** ein Keilwinkel (42) im Bereich zwischen 10° und 30° liegt.

7. Chirurgisches Messer nach Anspruch 5 oder 6, **dadurch gekennzeichnet, daß** ein Keilwinkel zwischen 15° und 25° liegt.

8. Chirurgisches Messer nach einem der vorangehenden Ansprüche, **dadurch gekennzeichnet, daß** eine Schneidkante (34) der Klinge (14) eine Welligkeit aufweist.

9. Chirurgisches Messer nach Anspruch 8, **dadurch gekennzeichnet, daß** eine mittlere Wellentiefe (40) im Bereich zwischen 0,03 mm und 0,16 mm liegt.

10. Chirurgisches Messer nach Anspruch 9, **dadurch gekennzeichnet, daß** eine mittlere Wellentiefe (40) im Bereich zwischen 0,06 mm und 0,10 mm liegt.

11. Chirurgisches Messer nach einem der Ansprüche 8 bis 10, **dadurch gekennzeichnet, daß** ein mittlerer Wellenabstand im Bereich zwischen 0,05 mm und 0,25 mm liegt.

12. Chirurgisches Messer nach Anspruch 11, **dadurch gekennzeichnet, daß** ein mittlerer Wellenabstand im Bereich zwischen 0,1 mm und 0,2 mm liegt.

13. Chirurgisches Messer nach einem der vorangehenden Ansprüche, **dadurch gekennzeichnet, daß** der Faseranteil im Kunststoffmaterial im Bereich zwischen 25 % und 70 % liegt.

14. Chirurgisches Messer nach Anspruch 13, **dadurch gekennzeichnet, daß** der Faseranteil im Kunststoffmaterial im Bereich zwischen 40 % und 60 % liegt.

15. Chirurgisches Messer nach einem der vorangehenden Ansprüche, **dadurch gekennzeichnet, daß** das Kunststoffmaterial durch Glasfasern verstärkt ist.

16. Chirurgisches Messer nach einem der vorangehenden Ansprüche, **dadurch gekennzeichnet, daß** das Kunststoffmaterial durch Kohlenstoffasern verstärkt ist.

17. Chirurgisches Messer nach einem der vorangehenden Ansprüche, **dadurch gekennzeichnet, daß** die Fasern (56) des Kunststoffmaterials an einer Schneidkante (34) der Klinge (14) im statistischen Mittel quer zur Schneidkante (34) ausgerichtet sind.

18. Chirurgisches Messer nach einem der vorangehenden Ansprüche, **dadurch gekennzeichnet, daß** die Fasern des Kunststoffmaterials in der Klinge (14) außerhalb eines Schneidkantenbereichs (52) im statistischen Mittel parallel oder in einem kleinen Winkel zu der Schneidkante (34) ausgerichtet sind.

19. Chirurgisches Messer nach einem der vorangehenden Ansprüche, **dadurch gekennzeichnet, daß** die Klinge (14) in einer Form (44) hergestellt ist, welche zur Herstellung der Schneidkante (34) im wesentlichen auf Null ausläuft.

20. Chirurgisches Messer nach einem der vorangehenden Ansprüche, **dadurch gekennzeichnet, daß** die Klinge (14) so hergestellt ist, daß flüssiger Kunststoff so in einer Form (44) geführt ist, daß sich Fasern (56) an einer Schneidkante (34) quer zu dieser ausrichten.

21. Chirurgisches Messer nach einem der vorangehenden Ansprüche, **dadurch gekennzeichnet, daß** eine mittlere Faserlänge in einem Bereich (52) der Klinge (14) an einer Schneidkante (34) kürzer ist als eine mittlere Faserlänge außerhalb des Bereichs (52) der Schneidkante (34).

22. Chirurgisches Messer nach einem der vorangehenden Ansprüche, **dadurch gekennzeichnet, daß** eine mittlere Faserlänge im Bereich (52) der Schneidkante (34) zwischen 20 µm und 50 µm liegt.

23. Chirurgisches Messer nach Anspruch 21 oder 22, **dadurch gekennzeichnet, daß** eine mittlere Faserlänge außerhalb des Bereichs (52) der Schneidkante (34) zwischen 70 µm und 200 µm liegt.

24. Chirurgisches Messer nach einem der vorangehenden Ansprüche, **gekennzeichnet durch** eine Ausbildung als Schälmesser (10).

25. Chirurgisches Messer nach einem der vorangehenden Ansprüche, **dadurch gekennzeichnet, daß** die Klinge (14) quer zu einer Längsrichtung (28) eines Haltegriffs (24) orientiert ist, wobei der Klingenhalter (12) im Bereich seines Halteendes (32) so bogenförmig ausgebildet ist, daß die Klinge (14) versetzt zu einer Längsebene (33) des Haltegriffs (24) angeordnet ist.

26. Chirurgisches Messer nach Anspruch 25, **dadurch gekennzeichnet, daß** eine Schneidkante (34) der Klinge (14) einem Ende (32) des Klingenhalters (12) abgewandt angeordnet ist.

27. Chirurgisches Messer nach einem der vorangehenden Ansprüche, **dadurch gekennzeichnet, daß** als Kunststoffmaterial ein faserverstärkter thermoplastischer Kunststoff eingesetzt wird.

## Claims

1. A surgical knife having a blade holder (12) and a blade (14), which blade (14) is manufactured from a fibre-reinforced plastics material.

2. A surgical knife according to claim 1, **characterised in that** the blade (14) is integrally arranged on the blade holder (12).

3. A surgical knife according to claim 1 or 2, **characterised in that** the blade holder (12) is arranged on a hand grip (24).

4. A surgical knife according to claim 3, **characterised in that** the hand grip (24) and the blade holder (12) are formed in one piece.

5. A surgical knife according to any one of the preceding claims, **characterised in that** the blade (14) is wedge-shaped at a cutting edge (34).

6. A surgical knife according to claim 5, **characterised in that** a wedge angle (42) lies in the range between 10° and 30°.

7. A surgical knife according to claim 5 or 6, **characterised in that** a wedge angle lies between 15° and 25°.

8. A surgical knife according to any one of the preceding claims, **characterised in that** a cutting edge (34) of the blade (14) is undulated.

9. A surgical knife according to claim 8, **characterised in that** an average undulation depth (40) lies in the range between 0.03 mm and 0.16 mm.

10. A surgical knife according to claim 9, **characterised in that** an average undulation depth (40) lies in the range between 0.06 mm and 0.10 mm.

11. A surgical knife according to any one of claims 8 to 10, **characterised in that** an average undulation spacing lies in the range between 0.05 mm and 0.25 mm.

12. A surgical knife according to claim 11, **characterised in that** an average undulation spacing lies in the range between 0.1 mm and 0.2 mm.

13. A surgical knife according to any one of the preceding claims, **characterised in that** the fibre content in the plastics material lies in the range between 25% and 70%.

14. A surgical knife according to claim 13, **characterised in that** the fibre content in the plastics material lies in the range between 40% and 60%.

15. A surgical knife according to any one of the preceding claims, **characterised in that** the plastics material is reinforced by glass fibres.

16. A surgical knife according to any one of the preceding claims, **characterised in that** the plastics material is reinforced by carbon fibres.

17. A surgical knife according to any one of the preceding claims, **characterised in that** at a cutting edge (34) of the blade (14), the fibres (56) of the plastics material are oriented, on a statistical average, transversely to the cutting edge (34)

18. A surgical knife according to any one of the preceding claims, **characterised in that** in the blade (14), outside of a cutting-edge region (52), the fibres of the plastics material are oriented, on a statistical average, parallel to or at a small angle to the cutting edge (34).

19. A surgical knife according to any one of the preceding claims, **characterised in that** the blade (14) is manufactured in a mould (44) which substantially tapers off to zero in order to manufacture the cutting edge (34).

20. A surgical knife according to any one of the preceding claims, **characterised in that** the blade (14) is manufactured in such a manner that a liquid plastics material is guided in a mould (44) in such a manner that, at a cutting edge (34), fibres (56) run transversely to the latter.

21. A surgical knife according to any one of the preceding claims, **characterised in that** an average fibre length in a region (52) of the blade (14) at a cutting edge (34) is shorter than an average fibre length outside of the region (52) of the cutting edge (34).

22. A surgical knife according to any one of the preceding claims, **characterised in that** an average fibre length in the region (52) of the cutting edge (34) lies between 20 µm and 50 µm.

23. A surgical knife according to claim 21 or 22, **characterised in that** an average fibre length outside of the region (52) of the cutting edge (34) lies between 70 µm and 200 µm.

24. A surgical knife according to any one of the preceding claims, **characterised by** a design as a paring knife (10).

25. A surgical knife according to any one of the preceding claims, **characterised in that** the blade (14) is directed transversely to a longitudinal direction (28) of a hand grip (24), the blade holder (12) being curved in the region of its holding end (32) in such a manner that the blade (14) is arranged out-of-line with regard to a longitudinal plane (33) of the hand grip (24).

26. A surgical knife according to claim 25, **characterised in that** a cutting edge (34) of the blade (14) is arranged remote from an end (32) of the blade holder (12).

27. A surgical knife according to any one of the preceding claims, **characterised in that** a fibre-reinforced thermoplastic plastics material is used as a plastics material.

## Revendications

1. Couteau chirurgical comprenant un support de lame de coupe (12) et une lame de coupe (14), qui est fabriquée en un matériau synthétique renforcé de fibres.

2. Couteau chirurgical selon la revendication 1, **caractérisé en ce que** la lame de coupe (14) est agencée d'un seul tenant sur le support de lame de coupe (12).

3. Couteau chirurgical selon la revendication 1 ou 2, **caractérisé en ce que** lie support de lame de coupe (12) est agencé sur une poignée (24).

4. Couteau chirurgical selon la revendication 4, **caractérisé en ce que** la poignée (24) et le support de lame de coupe (12) sont réalisés d'un seul tenant.

5. Couteau chirurgical selon l'une des revendications précédentes, **caractérisé en ce que** la lame de coupe (14) est réalisée en forme de coin au niveau d'un tranchant de coupe (34).

6. Couteau chirurgical selon la revendication 5, **caractérisé en ce qu'**un angle de coin (42) se situe dans une plage entre 10° et 30°.

7. Couteau chirurgical selon la revendication 5 ou 6, **caractérisé en ce qu'**un angle de coin se situe entre 15 et 25°.

8. Couteau chirurgical selon l'une des revendications précédentes, **caractérisé en ce qu'**un tranchant de coupe (34) de la lame de coupe (14) présente une ondulation.

9. Couteau chirurgical selon la revendication 8, **caractérisé en ce qu'**une profondeur moyenne d'onde (40) se situe dans une plage entre 0,03 mm et 0,16 mm.

10. Couteau chirurgical selon la revendication 9, **caractérisé en ce qu'**une profondeur moyenne d'onde (40) se situe dans une plage entre 0,06 mm et 0,10 mm.

11. Couteau chirurgical selon l'une des revendications 8 à 10, **caractérisé en ce qu'**une distance moyenne d'espacement d'ondes se situe dans une plage entre 0,05 mm et 0,25 mm.

12. Couteau chirurgical selon la revendication 11, **caractérisé en ce qu'**une distance moyenne d'espacement d'ondes se situe dans une plage entre 0,1 mm et 0,2 mm.

13. Couteau chirurgical selon l'une des revendications précédentes, **caractérisé en ce que** la proportion de fibres dans le matériau synthétique se situe dans une plage entre 25% et 70%.

14. Couteau chirurgical selon la revendication 13, **caractérisé en ce que** la proportion de fibres dans le matériau synthétique se situe dans une plage entre 40% et 60%.

15. Couteau chirurgical selon l'une des revendications précédentes, **caractérisé en ce que** le matériau synthétique est renforcé par des fibres de verre.

16. Couteau chirurgical selon l'une des revendications précédentes, **caractérisé en ce que** le matériau synthétique est renforcé par des fibres de carbone.

17. Couteau chirurgical selon l'une des revendications précédentes, **caractérisé en ce que** les fibres (56) du matériau synthétique sont orientées en moyenne statistique, au niveau d'un tranchant de coupe (34) de la lame de coupe (14), transversalement au tranchant de coupe (34).

18. Couteau chirurgical selon l'une des revendications précédentes, **caractérisé en ce que** les fibres du matériau synthétique sont orientées en moyenne statistique, dans la lame de coupe (14) en-dehors d'une zone de tranchant de coupe (52), parallèlement au tranchant de coupe (34) ou selon un faible angle par rapport à ce dernier.

19. Couteau chirurgical selon l'une des revendications précédentes, **caractérisé en ce que** la lame de coupe (14) est fabriquée dans un moule (44), qui se termine par un bord de fuite sensiblement nul pour réaliser le tranchant de coupe (34).

20. Couteau chirurgical selon l'une des revendications précédentes, **caractérisé en ce que** la lame de coupe (14) est fabriquée de manière telle, que du matériau synthétique liquide soit dirigé dans un moule (44) de manière à ce que des fibres (56) s'orientent, au niveau d'un tranchant de coupe (34), transversalement par rapport à celui-ci.

21. Couteau chirurgical selon l'une des revendications précédentes, **caractérisé en ce qu'**une longueur moyenne de fibres est plus courte dans une zone (52) de la lame de coupe (14) au niveau d'un tranchant de coupe (34), qu'une longueur moyenne de fibres à l'extérieur de la zone (52) du tranchant de coupe (34).

22. Couteau chirurgical selon l'une des revendications précédentes, **caractérisé en ce qu'**une longueur moyenne de fibres dans la zone (52) du tranchant de coupe (34), se situe entre 20 µm et 50 µm.

23. Couteau chirurgical selon la revendication 21 ou 22, **caractérisé en ce qu'**une longueur moyenne de fibres en-dehors de la zone (52) du tranchant de coupe (34), se situe entre 70 µm et 200 µm.

24. Couteau chirurgical selon l'une des revendications précédentes, **caractérisé par** une réalisation en tant que couteau racloir (10).

25. Couteau chirurgical selon l'une des revendications précédentes, **caractérisé en ce que** la lame de coupe (14) est orientée transversalement à une direction longitudinale (28) d'une poignée (24), le support de lame de coupe (12) étant réalisé selon une forme coudée dans la zone de son extrémité de maintien (32) de manière telle, que la lame de coupe (14) soit agencée de façon décalée par rapport à un plan longitudinal (33) de la poignée (24).

26. Couteau chirurgical selon la revendication 25, **caractérisé en ce qu'**un tranchant de coupe (34) de la lame de coupe (14) est agencé de manière à être dirigé à l'opposé d'une extrémité (32) du support de lame de coupe (12).

27. Couteau chirurgical selon l'une des revendications précédentes, **caractérisé en ce qu'**en guise de matériau synthétique on met en oeuvre une matière synthétique thermoplastique renforcée de fibres.
